# EUROPÄISCHE PATENTANMELDUNG

(11) **EP 1 754 506 A2**
(43) Veröffentlichungstag der Anmeldung: **21.02.2007**
(21) Anmeldenummer: 06012717.2
(22) Anmeldetag: 21.06.2006
(51) Int. Cl.: A61N 1/05

(54) **Vorrichtung zur Aufnahme einer chirurgischen Elektrode**

(30) Priorität: 18.08.2005 DE 102005038982
(71) Anmelder: CARDIOMEDICAL GmbH, 30855 Langenhagen (DE)
(72) Erfinder: Wiedenbein, Wolfgang, 30926 Seelze (DE)

(57) **Zusammenfassung**

Die vorliegende Erfindung bezieht sich allgemein auf das Gebiet der Medizin im chirurgischen Bereich. Im chirurgischen Bereich werden unter Anwendung der Elektrotherapie, chirurgische Instrumente, Vorrichtungen und Verfahren, um nichtmechanische Formen von Energie zum oder vom Körper weg zu transportieren, eingesetzt. Solche Instrumente sind zum Beispiel chirurgische Elektroden, so genannte Herzdrähte, insbesondere temporäre myokardiale Elektroden. Die Verwendung der temporären myokardialen Elektroden erfolgt unter Zuhilfenahme einer Vorrichtung. Die Vorrichtung ist zum Auf- und Abwickeln der chirurgischen Elektrode geeignet, wobei das Auf- und Abwickeln der temporären myokardialen Elektrode im postoperativen Verfahren durch ein erfindungsgemäßes Halteelement sicherer und leichter gestaltet wird.

## Beschreibung

### Technisches Gebiet

Die vorliegende Erfindung bezieht sich allgemein auf das Gebiet der Medizin im chirurgischen Bereich. Im chirurgischen Bereich werden unter Anwendung der Elektrotherapie, chirurgische Instrumente, Vorrichtungen und Verfahren, um nichtmechanische Formen von Energie zum oder vom Körper weg zu transportieren, eingesetzt. Solche Instrumente sind zum Beispiel chirurgische Elektroden, sogenannte Herzdrähte, insbesondere temporäre myokardiale Elektroden die der zeitweiligen Überwachung und/ oder Stimulation der Tätigkeit eines Herzens und somit der optimalen Therapie postoperativer Herzrhythmusstörungen dienen.

### Hintergrund im Stand der Technik

Die temporäre myokardiale Elektrode stellt die Verbindung zwischen dem Herzen und der elektromedizinischen Einrichtung, beispielsweise dem temporären Herzschrittmacher, den EKG-Monitoren oder dergleichen, zur zuverlässigen und effektiven temporären Stimulation der Herzmuskelnzellen dar. Die temporäre myokardiale Elektrode (nachstehend als TME bezeichnet), besteht daher im Prinzip zur Befestigung am Herzen an dem distalen Ende, aus einer gebogenen chirurgischen Nadel, mit der an einer entsprechenden Stelle in die Herzwand eingestochen werden kann. Zu der TME gehört ferner ein nach außen durch den Thorax zu führendes proximales Ende, welches, bevor es an eine elektromedizinische Einrichtung angeschlossen wird, ebenfalls mit einer gebogenen chirurgischen Nadel ausgestattet ist. Das distale und proximale Ende der Elektrode ist im Prinzip aus einem flexiblen elektrischen Leiter, der mit einer Isolationsschicht umgeben ist, verbunden. Derartige TM- Elektroden gibt es in unipolarer, bipolarer, biventricularer oder quadripolarer Ausführung. Zur Positionierung der TM-Elektroden am Herzen können diese noch mit verschiedenen Fixierungsmitteln, wie Zick- Zack Biegung, Anker, Schleife oder atraumatischer Fixierung ausgestattet sein.

Solche chirurgischen Elektroden sind aus dem Stand der Technik hinreichend bekannt, siehe beispielsweise Produktkatalog unter " Osypka Temporary Myocardial Leads" , www. osypka.de oder www.vascomed.de/catalog.

Die TM- Elektrode ist zur leichteren Handhabung durch den Chirurgen vor der Benutzung steril verpackt und auf einem Trägermittel aufgewickelt. Nach dem operativen herzchirurgischen Eingriff wird die TM- Elektrode vom Chirurgen vom Trägermittel abgewickelt, wobei das Trägermittel aus einem rechteckigen flächigen Kunststoffkörper, der mit Befestigungselementen für die chirurgischen Nadeln ausgebildet ist, besteht. Das distale Ende der Leitung mit der chirurgischen Nadel wird vom Chirurg im Myokardium verankert, nachdem das proximale Teil der Elektrode von Innen durch die Brust nach außen verlegt wurde. Das proximale Teil der TM-Elektrode liegt nun lose und außerhalb des Patienten.

Die Längen der TM- Elektroden betragen idR. 200 cm, 220 cm oder 250 cm, wobei das proximale Ende nach Abschneiden bzw. Abbrechen der Thoraxnadel durch ein Adaptersystem oder Steckeranschlüsse mit der externen elektromedizinischen Einrichtung oder einem Verlängerungskabel verbunden wird.

Nachteil dieser Ausführungsform von Trägermitteln für chirurgische Elektroden ist das umständliche Abwickeln der TME vor der Implantation und das ebenfalls, bei Verlegung des Patienten, beispielsweise aus dem OP- Bereich in den Intensivbereich, umständliche Aufwickeln der TME auf das relativ große Trägermittel. Aus Sicherheitsgründen ist ein Aufwickeln der TM-Elektrode aber notwendig, um Verletzungen des Patienten nach dem operativen Eingriff durch unbeabsichtigten Zug an der herumliegenden TM- Elektrode zu vermeiden.

In einer weiteren, aus dem Stand der Technik bekannten chirurgischen Elektrode ist eine Befestigungsvorrichtung für die chirurgische Elektrode aus der DE 199 59 655 B4 bekannt. Die Befestigungsvorrichtung kann außerhalb des Körpers des Patienten mit dessen Haut verklebbar oder vernähbar verbunden werden. Der Nachteil dieser Befestigungsvorrichtung besteht darin, dass diese nicht zum einfachen Aufwickeln der TM- Elektrode geeignet ist, sondern nur als Adapter für ein Verlängerungskabel zum externen Herzschrittmacher dient.

Die Firma Medical Concepts als Hersteller von temporären bipolaren myokardialen Herzdrähten hat Trägermittel aus dem Stand der Technik weiterentwickelt und das Trägermittel zur Aufnahme der TM- Elektrode, ähnlich einer Garnrolle, ausgebildet. Das Trägermittel ist eine Vorrichtung zum Auf- und Abwickeln einer chirurgischen Elektrode, bestehend aus mehreren Bauteilen, mit einem Trägerteil, welches aus zwei parallel zueinander flächigen Scheiben, die kreisrund ausgeführt sein können, besteht, wobei die runden flächigen Scheiben eben und mit einer Bohrung in der Mitte versehen sind. Die beiden runden flächigen Scheiben sind im Bereich der Bohrung durch einen Hohlzylinder miteinander verbunden. Das Trägerteil bildet somit einen so genannten Spulenkörper, der ein- oder mehrteilig ausgeführt sein kann. Durch die Bohrung des Hohlzylinders des Spulenkörpers wird eine Achse geschoben, wobei zwischen der Bohrung und der Achse ein geringer Luftspalt besteht, sodass der Spulenkörper drehbar auf der Achse gelagert ist. Die Achse besitzt an dem einen Ende einen Rezess als Anschlag für eine Seitenwand des Spulenkörpers. An dem anderen Ende der Achse befindet sich eine Bohrung in Längsrichtung der Achse, die als Sackbohrung ausgeführt und in die eine Verschlussscheibe mit Nabe eingesteckt werden kann. Die Sackbohrung der Achse und der Durchmesser der Nabe der Verschlussscheibe sind als Presspassung zueinander ausgelegt. Die Verschlussscheibe bildet den Anschlag für die andere Seitenwand des Spulenkörpers. Zwischen dem Rezess der Achse und der Verschlussscheibe ist der Spulenkörper zum Auf- und Abwickeln der chirurgischen Elektrode angeordnet. Die Scheiben bzw. dessen Seitenwände des Spulenkörpers sind Träger mindestens eines Befestigungsmittels zur Aufnahme der chirurgischen Elektroden, wobei das Befestigungsmittel aus einem Hartschaumpunkt gebildet ist, vorzugsweise sind zwei Hartschaumpunkte angebracht. Die Anbringung der Befestigungsmittel erfolgt durch selbstklebende Hartschaumpunkte.

Diese Bauform des Trägerteils für chirurgische Elektroden weist aber mehrere gravierende Nachteile beim Handling, der Reinigung und Sterilisation auf. Der Zwischenraum, der sich durch die beiden runden zueinander beabstandeten Scheiben des Spulenkörpers, aufgrund der relativ geringen aufzuwickelnden Länge und des geringen Durchmessers der TME ergibt, ist sehr gering. Soll die TME durch den Chirurgen, zum Beispiel wegen Verlegung des Patienten, wieder auf die Vorrichtung aufgewickelt werden, muss der Chirurg den Spulenkörper in eine Hand nehmen und mit der anderen Hand die TME aufwickeln. Aufgrund der geringen Beabstandung der Seitenwände des Spulenkörpers verschließen jedoch die Finger, die den Spulenkörper halten, die kreisrunde Öffnung des Spulenkörpers, sodass das Aufwickeln sehr umständlich, schwierig und fehlerbehaftet erfolgt. Die Gefahr, den Patienten durch ungewollten Zug an der TME zu verletzen, ist aufgrund des schlechten Handlings der Vorrichtung leicht gegeben. Des weiteren ist es umständlich, aufgrund fehlender Öffnungen in den Scheiben, die TME im Spulenkörper einfach aufzuwickeln, da die Seitenwände des Spulenkörpers geschlossen sind. Eine den medizinischen Anforderungen (EU-Richtlinie) entsprechende Reinigung und Sterilisation der Vorrichtung von Partikeln ist ebenfalls nicht möglich.

Der Erfindung liegt daher die Aufgabe zugrunde, eine Vorrichtung zum Auf- und Abwickeln für temporäre myokardiale Elektroden der eingangs genannten Art zu schaffen, welche die vorgenannten Nachteile der bekannten Anordnungen vermeidet und eine technische Lösung anzugeben, die es ermöglicht, eine kostengünstigere, mit einfacher Funktionsgeometrie ausgestattete Vorrichtung für chirurgische Elektroden mit erhöhten Ansprüchen zu schaffen, welche eine Bauform aufweist, die für viele Ausführungsformen von temporären myokardialen Elektroden zum Auf- und Abwickeln geeignet ist.

Erfindungsgemäß wird dieses Problem durch die kennzeichnenden Merkmale des Patentanspruchs 1 gelöst. Vorteilhafte Ausgestaltungen und Weiterbildungen der Erfindung ergeben sich aus den nachstehenden Unteransprüchen und den nachfolgenden Beschreibungen

### Beschreibung der Erfindung

Um eine mit diesen Merkmalen der vorliegenden Erfindung ausgestattete Vorrichtung für chirurgische Elektroden, insbesondere temporäre myokardiale Elektroden für die postoperative Herzchirurgie zu schaffen, wird erfindungsgemäß vorgeschlagen, eine Vorrichtung herzustellen, die das Handling für den Chirurgen wesentlich erleichtert, erhöhte Sicherheitsbedingungen erfüllt und eine wesentlich bessere Reinigung und Sterilisation ermöglicht. Diese Aufgabe wird erfindungsgemäß dadurch gelöst, dass die Vorrichtung zum Auf- und Abwickeln einer chirurgischen Elektrode, bestehend aus mehreren Bauteilen, zusätzlich mit einem Halteelement, mit Öffnungen im Trägerteil und einem Verbindungselement ausgestattet ist.

Die Benutzung der erfindungsgemäßen Vorrichtung erfolgt im praktischen Einsatz wie nachstehend beschrieben.

Nach dem operativen herzchirurgischen Eingriff, wird zur Verbindung des Herzens mit der elektromedizinischen Einrichtung, vorzugsweise einem temporären postoperativen atrialen und ventrikularen Schrittmacher, die temporäre myokardiale Elektrode durch den Chirurgen von der Vorrichtung abgewickelt. Hierzu wird die Vorrichtung der sterilen Verpackung entnommen und zwischen den Daumen und Zeigefinger einer Hand geklemmt, wobei der Daumen auf das Ende der Nabe und der Zeigefinger auf die Verschlussscheibe, oder umgekehrt, greift. Dadurch ist das Trägermittel drehbar zwischen dem Daumen und dem Zeigefinger angeordnet. Nachdem die am distalen Ende der TM- Elektrode befestigte chirurgische Nadel (Myokardnadel) mit der anderen Hand aus dem Befestigungsmittel entnommen wurde, lässt sich durch einfaches Ziehen an der TME diese vom Trägerteil abwickeln. Danach erfolgt die Entnahme der am proximalen Ende angeordneten chirurgischen Nadel (Thoraxnadel) aus dem Befestigungsmittel. Das Abwickeln der TME von der Vorrichtung kann auch in umgekehrter Reihenfolge durchgeführt werden. Die Befestigungsmittel für die chirurgischen Nadeln sind an einer der Außenflächen der Scheiben des Trägerteils angeordnet.

Das Abwickeln der TME kann auch nach einem weiteren Verfahren erfolgen. Hierzu wird das erfindungsgemäße Halteelement von der Vorrichtung, bzw. von dem Trägerteil abgeschwenkt und von Daumen und Zeigefinger einer Hand erfasst. Das Abwickeln erfolgt dann wie zuvor beschrieben. Ist die TME von der Vorrichtung abgewickelt, liegen zwei getrennte Gegenstände vor. Einerseits die TME, die, wie zuvor beschrieben, im postoperativen Eingriff verwendet wird und andererseits die Vorrichtung, die zur späteren Wiederverwendung idR. auf dem OP-Tisch aufbewahrt wird. Zur sicheren Aufbewahrung der Vorrichtung ist an der Außenfläche der Scheibe des Trägerteils, die der Scheibe mit den Befestigungsmitteln gegenüberliegt, ein Verbindungselement angeordnet.

Das Verbindungselement besteht aus einem Klebeband, welches beidseitig mit einem Haftkleber versehen ist. Dieses doppelseitige Klebeband stellt das Verbindungselement zwischen der Vorrichtung und beispielsweise der Haut eines Patienten dar. Die eine Seite des Verbindungselementes ist dazu auf einer Außenfläche einer Scheibe des Trägermittels aufgeklebt, während die andere Seite des Verbindungselementes auf jeglichen Flächen im Bereich des Patienten oder des Krankenbettes befestigt werden kann, sodass die Vorrichtung nicht lose herumliegt oder verloren geht. Bei der Verlegung des Patienten aus dem Operationssaal auf die Intensivstation es notwendig, dass der Chirurg die TME vorher auf die Vorrichtung aufwickelt. Für den Chirurgen ist es ein Einfaches, die erfindungsgemäße Vorrichtung vom Aufbewahrungsort, beispielsweise dem Operationstisch, aufzunehmen, die TME aufzuwickeln und im aufgewickelten Zustand mit dem elektromedizinischen Gerät wieder an einer geeigneten Stelle des Patienten oder des Krankenbettes zu fixieren.

Das Handling beim Aufwickeln der TME wird durch das erfindungsgemäße Halteelement wesentlich sicherer und das Aufwickeln ist für den Chirurgen leichter durchzuführen. Hierzu braucht das Halteelement aus Kunststoff, welches ring- oder stabförmig ausgebildet und beweglich an der Vorrichtung angeordnet ist, nur durch eine Schwenkbewegung vom Trägermittel ausgeklappt zu werden. Der Daumen und der Zeigefinger einer Hand ergreifen das Halteelement, während die andere Hand die TME auf das Trägerteil aufwickelt. Zu Beginn des Aufwickelvorganges wird zum vorteilhaften Aufwickeln der TME diese durch einen erfindungsgemäßen Schlitz geführt, der in einer Scheibe des Trägerteils eingelassen ist, vorzugsweise sind zwei sich um 180 Grad gegenüberliegende Schlitze angeordnet. Aufgrund der erfindungsgemäßen Vorrichtung ist der Aufwickelvorgang der TME sicher und dauert nur wenige Sekunden. Der Aufwickelvorgang der TME ist beendet, wenn die Vorrichtung einerseits relativ in der Nähe der Austrittsstelle der TME, die aus dem Thorax heraus führt und andererseits der temporäre Herzschrittmacher sich in der Nähe der Vorrichtung befindet, sodass beispielsweise beim Transport des Patienten der lange elektrische Leiter der TME nirgends störend herumliegt und eine Verletzung des Patienten somit ausgeschlossen wird.

Ein Ausführungsbeispiel der Erfindung ist in den Zeichnungen rein schematisch dargestellt und wird nachfolgend näher beschrieben. Es zeigt
- Figur 1: in einer auseinandergezogenen perspektivischen Darstellung eine Vorrichtung zur Aufnahme einer chirurgischen Elektrode und
- Figur 2: eine Schnittansicht einer komplettierten Vorrichtung für temporäre myokardiale Elektroden und
- Figur 3: eine Vorrichtung mit einem alternativen Halteelement.

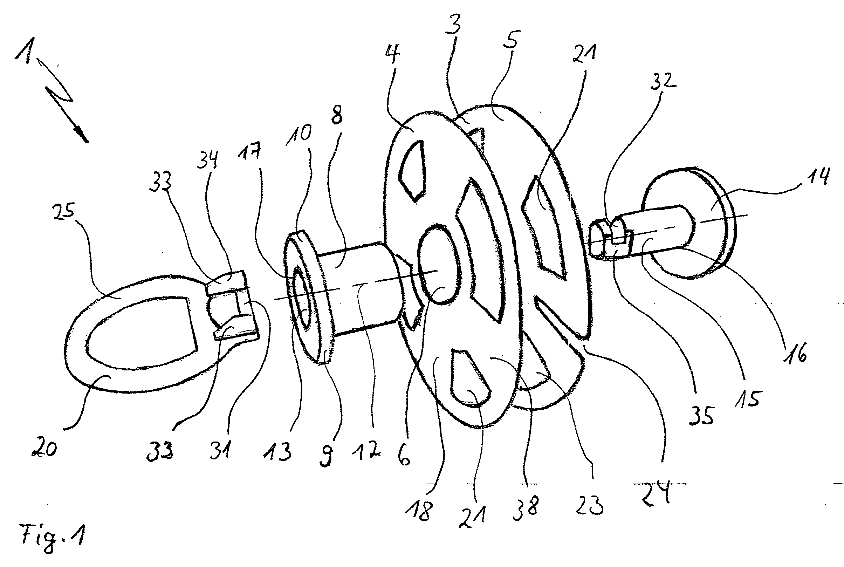

In der Fig.1 ist in einer auseinandergezogenen perspektivischen Darstellung eine Vorrichtung zum Auf- und Abwickeln einer chirurgischen Elektrode 2, insbesondere einer temporären myokardialen Elektrode 2 (nicht dargestellt), bestehend aus mehreren Bauteilen, aufgezeigt, wobei die Bauteile aus einem Trägerteil 3, einer Achse 8, einer Verschlussscheibe 14, einem Befestigungsmittel 19, einem Halteelement 20 und einem Verbindungselement 22 bestehen.

Das Trägerteil 3, die Achse 8, die Verschlussscheibe 14 und das Halteelement 20 bestehen aus Kunststoff. Andere Materialien wie beispielsweise Metall können zur Herstellung der Vorrichtung ebenfalls verwendet werden.

Das Trägerteil 3 aus Kunststoff wird aus zwei parallel zueinander stehenden Scheiben 4,5, die eine Bohrung 6 enthalten, gebildet, wobei die beabstandeten Scheiben 4,5 durch einen Hohlzylinder 7 miteinander verbunden sind. Das Trägerteil 3 kann ein- oder mehrteilig ausgeführt sein. Erfindungsgemäß enthält das Trägerteil 3 Öffnungen 21. Die Öffnungen 21 werden aus Durchbrüchen 23 und Schlitzen 24 gebildet, die in den Scheiben 4,5 des Trägerteils 3 angeordnet sind. Vorzugsweise werden vier Durchbrüche 23 im Abstand von jeweils 90 Grad vorgesehen, wobei auch eine andere Anzahl von Durchbrüchen 23, beispielsweise drei oder fünf Durchbrüche 23 denkbar, sind. Die Durchbrüche 23 erleichtern das medizinisch notwendige Reinigen und Sterilisieren. Dieses dient der Reduzierung von Fremdpartikeln in der Vorrichtung, um eine Kontamination und deren Auswirkungen beim Patienten wesentlich zu verringern bzw. zu vermeiden. Zwischen den beabstandeten Durchbrüchen 23 ergeben sich Speichen 38, die der Befestigung der Befestigungsmittel 19 dienen. Während die Schlitze 24 in der Scheibe 5 das Einfädeln der aufzuwickelnden TME 2 wesentlich erleichtern und somit ein einfacheres Handling ermöglichen.

Die Achse 8 besitzt an einem Achsenende 9 einen Rezess 10 und ist in Längsrichtung der Mittellinie 12 der Achse 8 mit einer durchgehend verlaufenden Bohrung 13 versehen. In die Bohrung 13 der Achse 8 wird die Nabe 15 der Verschlussscheibe 14 eingeführt, wobei der Durchmesser 16 der Nabe 15 dem Durchmesser 17 der Bohrung 13 der Achse 8 entspricht. Die beiden Durchmesser 16,17 bilden eine Presspassung. Es besteht auch die Möglichkeit, die beiden Durchmesser 16,17 mit einer Spielpassung auszustatten.

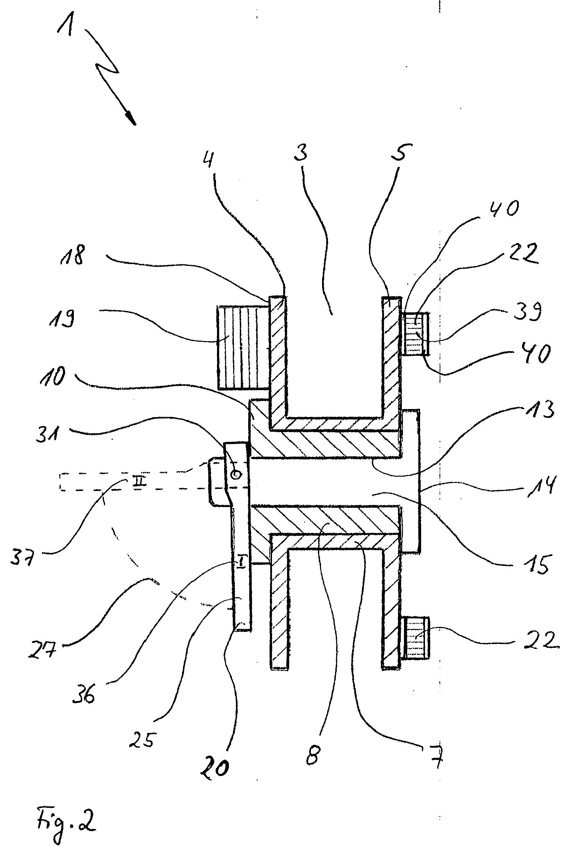

An der Außenfläche 18 der Scheibe 4 ist ein Befestigungsmittel 19 (siehe Figur 2) angeordnet. Das Befestigungsmittel 19 dient der Aufnahme der chirurgischen Elektroden und besteht aus mindestens einem Hartschaumpunkt, vorzugsweise zwei Hartschaumpunkten. Auch ist es denkbar, anstelle der Hartschaumpunkte andere Befestigungsmittel 19, die der Aufnahme der chirurgischen Nadeln zur Aufbewahrung dienen, zu verwenden. Beispielsweise sind Anordnungen von Kunststofferhöhungen aus Noppen, Stiften oder ähnlichem an einer der Außenseitenflächen 18 der Scheiben 4,5 des Trägerteils 3, welche die chirurgischen Nadeln klemmen können, möglich.

Das Halteelement 20 ist ringförmig 25 ausgebildet und an der Vorrichtung 1 beweglich angeordnet, wobei die Bewegung des Halteelementes 20 eine Schwenkbewegung 27 darstellt. Das ringförmige 25 Halteelement 20 ist an der Nabe 15 der Verschlussscheibe 15 angeordnet. Die Anordnung des Halteelementes 20 und der Nabe 15 besteht aus einer lösbaren Verbindung 30. Die lösbare Verbindung 30 zwischen dem ringförmigen 25 Halteelement 20 und der Nabe 15 wird durch einen am Halteelement 20 angeordneten Steg 31 und einen in der Nabe 15 angeordneten Querschlitz 32 gebildet. Der Steg 31 des Halteelements 20 ist in den Querschlitz 32 der Nabe 15 einsetzbar. Damit das Halteelement 20 eine auf einem Kreisbogen geradlinige Schwenkbewegung 27 ausführen kann, sind am ringförmigen 25 Halteelement 20 im Randbereich des Steges 31 zur Führung der Schwenkbewegung 27 des Halteelementes 20 zwei seitliche Führungen 33 angeordnet. Diese seitlichen Führungen 33 sind parallel zueinander beabstandet und stehen senkrecht zum Steg 31. Eine entsprechende Anordnung an der Nabe 15 der Verschlussscheibe 14 hat zwei beabstandete Flächen 35 im Bereich des Querschlitzes 32. Die Flächen 34 der Führungen 33 liegen somit an den zwei parallel beabstandeten Flächen 35 der Nabe 15 an, die im Bereich des Querschlitzes 32 der Nabe 15 angeordnet sind. Das ringförmige 25 Halteelement 20 kann somit aus der anliegenden Stellung I (siehe Figur 2), wobei das ringförmige 25 Halteelement 20 parallel zur Scheibe 4 des Trägerteils 3 anliegt, vom Benutzer um 90 Grad abgeschwenkt werden, wobei die abgeschwenkte Stellung des ringförmigen 25 Halteelementes 20 der senkrechten Stellung II (gestrichelte Darstellung Fig.2) des Halteelementes 20 zur Scheibe 4 der Vorrichtung 1 entspricht.

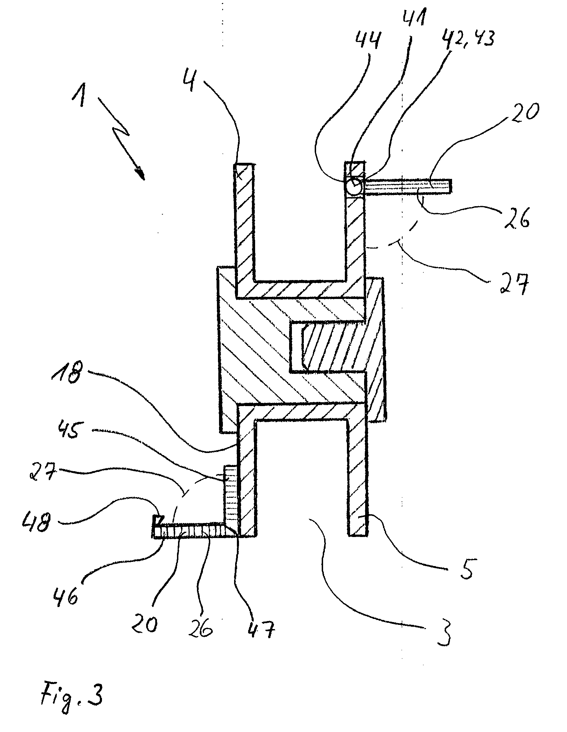

Das Halteelement 20 kann auch stabförmig 26 ausgebildet und beweglich an der Vorrichtung 1 angeordnet sein, wobei das stabförmige 26 Halteelement 20 ebenfalls wie das ringförmige 25 Halteelement 20 eine Schwenkbewegung ausführt. Das stabförmige 26 Halteelement 20 ist hingegen dem ringförmigen 25 Halteelement 20 nicht an der Nabe 15 der Verschlussscheibe 14 angeordnet, sondern an der Außenfläche 18 der Scheibe 4 des Trägerteils 3 (siehe Fig.3).

Das Befestigungsmittel 19 und das Verbindungselement 22 werden in Figur 2 näher beschrieben und entsprechend dargestellt.

Aus der Figur 2 ist eine komplettierte Vorrichtung 1 in Schnittdarstellung ersichtlich. Die komplettierte Vorrichtung 1 besteht aus dem Trägerteil 3 mit Hohlzylinder 7, im Hohlzylinder 7 befindet sich die eingeschobenen Achse 8 die drehbar gelagert ist und mit ihrem Rezess 10 an der Scheibe 4 anliegt. In der Bohrung 13 der Achse 8 befindet sich die Nabe 15 der Verschlussscheibe 14, wobei die Nabe 15 und die Verschlussscheibe 14 einteilig ausgebildet sind und die Verschlussscheibe 14 an der Scheibe 5 anliegt. Am Ende der Nabe 15, der Verschlussscheibe 14 gegenüberliegend, ist das ringförmige 25 Halteelement 20 durch Verbindungselemente 31,32 angeordnet. Dem Halteelement 20 kommt somit eine weitere Funktion, das Zusammenhalten der Bauteile (3, 8,14 u.15), zu.

Die Befestigungsmittel 19 für die chirurgischen Nadeln 2 (nicht dargestellt) sind variabel an der Außenfläche 18 der Scheibe 4 angeordnet, uzw. auf den Speichen 38. Die Speichen 38 werden durch die beabstandeten Öffnungen 21 gebildet. Die Außenflächen 18 der Speichen 38 sind die Träger für die Befestigungsmittel 19, vorzugsweise den Hartschaumpunkten und den Verbindungselement 22, wobei das Befestigungsmittel 19 auch an der Scheibe 5 und das Verbindungselement 22 an der Scheibe 4 angeordnet sein können. Das Verbindungselement 22 ist kreisringförmig ausgebildet und besteht aus einem Träger 39, der beidseitig mit einem Haftkleber 40 versehen ist, vorzugsweise einem doppelseitigen Klebeband.

Figur 3 beschreibt eine Vorrichtung1, bei der das Halteelement 20 nicht an der Nabe 15 der Verschlussscheibe 14 angeordnet ist, sondern an der Scheibe 4,5 des Trägerteils 3. Das Halteelement 20 ist stabförmig 26 ausgebildet und beweglich angeordnet. Die Bewegung des stabförmigen 26 Halteelements 20 entspricht einer Schwenkbewegung 27. In einer ersten Ausführung ist das stabförmige 26 Halteelement 20 einstückig und mit einem Kugelkopf 42 als Gelenk 41 ausgebildet, welcher in eine Bohrung 44 der Speiche 38 der Scheibe 4,5 eingeclipst werden kann, wobei vorzugsweise anstelle des Kugelkopfes 42 auch eine zylindrische Form 43 als Gelenk 41 zur Ausführung der Schwenkbewegung 27 des stabförmigen 26 Halteelements 20 in Frage kommt. Das in Form eines Zylinders 43 ausgeführte Gelenk 41 ist an einem Ende des stabförmigen 26 Halteelements 20 angeordnet und steht senkrecht zu diesem. In einer weiteren Ausführung ist das stabförmige 26 Halteelement 20 derart gestaltet, das dieses an der Außenfläche 18 der Speiche 38 befestigt ist. Das stabförmige 26 Halteelement 20 ist einstückig ausgeführt, besteht im Prinzip aber aus zwei Teilen 45,46 die durch ein Scharnier, vorzugsweise Filmscharnier verbunden sind. Im Endbereich des Teils 46 befindet sich ein Rastelement 48, beispielsweise bestehend aus einer Rastnase (nicht dargestellt), welche in ein im Teil 45 angeordnetes Gegenstück leicht einrasten kann. Das stabförmige 26 Halteelement 20 bzw. das Teil 45 des Halteelements 20 kann auf unterschiedlicher Weise an der Speiche 38 der Scheibe 4,5 des Trägerteils 3 befestigt werden, vorzugsweise durch verkleben. Die stabförmigen 26 Halteelemente 20 können beidseitig an der Vorrichtung 1 befestigt sein. Bei der Verwendung von zwei stabförmigen 26 Halteelementen 20 an einer Vorrichtung 1, sind diese um 180 Grad zueinander versetzt angeordnet.

### Bezugszeichenliste

- 1: Vorrichtung
- 2: chirurgische Elektrode
- 3: Trägerteil
- 4: Scheibe
- 5: Scheibe
- 6: Bohrung
- 7: Hohlzylinder
- 8: Achse
- 9: Achsenende
- 10: Rezess
- 11: ---
- 12: Mittellinie
- 13: Bohrung (Achse)
- 14: Verschlußscheibe
- 15: Nabe
- 16: Durchmesser (Nabe)
- 17: Durchmesser (Bohrung)
- 18: Außenfläche
- 19: Befestigungsmittel
- 20: Halteelement
- 21: Öffnungen
- 22: Verbindungselement
- 23: Durchbrüche
- 24: Schlitze
- 25: ringförmig
- 26: stabförmig
- 27: Schwenkbewegung
- 28: ---
- 29: ---
- 30: Verbindung
- 31: Steg
- 32: Querschlitz
- 33: Führungen (zu20)
- 34: Führungsflächen(zu20)
- 35: Führungsflächen(zu15)
- 36: Stellung I
- 37: Stellung II
- 38: Speiche
- 39: Träger
- 40: Haftkleber
- 41: Gelenk
- 42: Kugelkopf
- 43: Zylinderform
- 44: Bohrung (zu 4,5)
- 45: Teil I
- 46: Teil II
- 47: Scharnier
- 48: Rastelement

## Patentansprüche

1. Vorrichtung (1) zum Auf- und Abwickeln einer chirurgischen Elektrode (2), bestehend aus mehreren Bauteilen
- mit einem Trägerteil (3), das zwei parallel zueinander stehende Scheiben (4 ,5),die eine Bohrung (6) und Öffnungen (21) enthalten, umfasst, wobei die Scheiben (4,5) durch einen Hohlzylinder (7) verbunden sind und das Trägerteil (3) ein- oder mehrteilig ausgebildet ist,
- mit einer Achse(8), das an einem Achsenende (9) aus einem Rezess (10) und einer in Längsrichtung der Mittellinie (12) der Achse (8) verlaufenden Bohrung (13) gebildet wird,
- mit einer Verschlussscheibe (14) mit Nabe (15), wobei der Durchmesser (16) der Nabe (15) dem Durchmesser (17) der Bohrung (13) der Achse (8) entspricht,
- mit mindestens einem, an der Außenfläche (18) der Scheibe (4,5) angeordneten Befestigungsmittel (19) zur Aufnahme der chirurgischen Elektrode (2), **dadurch gekennzeichnet, dass** die Vorrichtung (1) durch ein Halteelement (20) und einem Verbindungselement (22) gebildet wird.

2. Vorrichtung nach Anspruch 1, **dadurch gekennzeichnet, dass** das Halteelement (20) ringförmig (25) oder stabförmig (26) ausgebildet ist.

3. Vorrichtung nach Anspruch 1 bis 2, **dadurch gekennzeichnet, dass** das Haltelement (20) beweglich an der Vorrichtung (19) angeordnet ist.

4. Vorrichtung nach Anspruch 1 bis 3, **dadurch gekennzeichnet, dass** die Bewegung des Halteelements (20) eine Schwenkbewegung darstellt.

5. Vorrichtung nach Anspruch 1 bis 4, **dadurch gekennzeichnet, dass** das ringförmige (25) Halteelement (20) an der Nabe (15) der Verschlussscheibe (14) angeordnet ist.

6. Vorrichtung nach Anspruch 1 bis 5, **dadurch gekennzeichnet, dass** zwischen dem ringförmigen (25) Halteelement (20) und der Nabe (15) der Verschlussscheibe (14) eine lösbare Verbindung (30) besteht.

7. Vorrichtung nach Anspruch 1 bis 6, **dadurch gekennzeichnet, dass** die lösbare Verbindung (30) des ringförmigen (25) Halteelements (20) mit der Nabe (15) durch einen am Halteelement (20) angeordneten Steg (31) und einen in der Nabe (15) angeordneten Querschlitz gebildet wird.

8. Vorrichtung nach Anspruch 1 bis 7, **dadurch gekennzeichnet, dass** am ringförmigen Halteelement (20) im Randbereich des Steges (31) zur Führung der Schwenkbewegung (27) des Halteelementes (20) zwei seitliche Führungen (33) angeordnet und parallel beabstandet sind und durch eine entsprechende Anordnung an der Nabe (15) der Verschlussscheibe (14) zwei parallel beabstandeten Flächen (35) im Bereich des Querschlitzes (32).

9. Vorrichtung nach Anspruch 1 bis 4, **dadurch gekennzeichnet, dass** das stabförmige (26) Halteelement (20) an der Scheibe (4,5) des Trägerteils (3) angeordnet ist.

10. Vorrichtung nach Anspruch 9, **dadurch gekennzeichnet, dass** das stabförmige (26) Halteelement (20) mit einem Gelenk (41) ausgebildet ist.

11. Vorrichtung nach Anspruch 1 bis 3, **dadurch gekennzeichnet, dass** das Gelenk (41) in die Bohrung (44) der Speiche (38) einclipsbar ist.

12. Vorrichtung nach einem der vorhergehenden Ansprüche, **dadurch gekennzeichnet, dass** das Trägerteil (3) Öffnungen (21) enthält.

13. Vorrichtung nach Anspruch 12, **dadurch gekennzeichnet, dass** die Öffnungen (21) im Trägerteil (3) aus Durchbrüchen (23) und schlitzen (24) gebildet sind.

14. Vorrichtung nach einem der vorhergehenden Ansprüche, **dadurch gekennzeichnet, dass** das Verbindungselement (22) an der Außenfläche (18) der Scheibe (5) des Trägerelementes (3) angeordnet ist.

15. Vorrichtung nach Anspruch 14, **dadurch gekennzeichnet, dass** das Verbindungselement (22) aus einem Träger (39), welcher beidseitig mit einem Haftkleber (40) ausgestattet ist, besteht.

16. Vorrichtung nach Anspruch 14 bis 15, **dadurch gekennzeichnet, dass** das Verbindungselement (22) aus einem doppelseitigen Klebeband besteht.

17. Vorrichtung nach Anspruch 14 bis 16, **dadurch gekennzeichnet, dass** das Verbindungselement (22) eine mit der Haut des Patienten verbindbare, zum Beispiel verklebbare oder vemähbare Befestigung eingeht.
